# EUROPEAN PATENT APPLICATION

(11) **EP 4 763 970 A1**
(43) Date of publication of application: **24.06.2026**
(21) Application number: 24383449.6
(22) Date of filing: 23.12.2024
(51) Int. Cl.: C12M 3/06, C12M 1/00, C12M 1/26

(54) **CELL CULTURE DEVICE**

(71) Applicant: LEARTIKER S. COOP., 48270 Markina-Xemein, Bizkaia (ES); DONOSTIA INTERNATIONAL PHYSICS CENTER (DIPC), 20018 Donostia, Gipuzkoa (ES); UNIVERSIDAD DEL PAÍS VASCO / EUSKAL HERRIKO UNIBERTSITATEA (UPV/EHU), 48940 Leioa (Bizkaia) (ES); ADMINISTRACIÓN GENERAL DE LA COMUNIDAD AUTÓNOMA DE EUSKADI, 01010 Vitoria-Gasteiz (Álava) (ES); CIBER-CENTRO DE INVESTIGACIÓN BIOMÉDICA EN RED, 28029 Madrid (ES)
(72) Inventor: ZALDUA HUICI, Ane Miren, E-48270 Markina-Xemein, Bizkaia (ES); ETXEBERRIA AIZPURU, Leire, E-48270 Markina-Xemein, Bizkaia (ES); ASTIGARRAGA BERGARA, Malen, E-48270 Markina-Xemein, Bizkaia (ES); COSSÍO MORA, Fernando P., E-20018 Donostia/San Sebastián (Guipuzcoa) (ES); RIVILLA DE LA CRUZ, Iván, E-20018 Donostia/San Sebastián (Guipuzcoa) (ES); PERUGORRIA MONTIEL, María Jesús, E-20014 Donostia - San Sebastián, Gipuzkoa (ES); FERNÁNDEZ DE PIÉROLA, Luis Bujanda, E-20014 Donostia - San Sebastián, Gipuzkoa (ES); BUQUÉ GARCÍA, Xabier, E-48940 Leioa, Bizkaia (ES); ASPICHUETA CELAÁ, Patricia, E-48940 Leioa (ES); BAÑALES ASURMENDI, Jesus María, E-20014 Donostia - San Sebastián, Gipuzkoa (ES); RODRIGUES, Pedro, E-20014 Donostia - San Sebastián, Gipuzkoa (ES); IZQUIERDO SÁNCHEZ, Laura, E-20014 Donostia - San Sebastián, Gipuzkoa (ES); LAPITZ, Ainhoa, E-20014 Donostia - San Sebastián, Gipuzkoa (ES); OLAIZOLA REBÉ, Irene, E-20014 Donostia - San Sebastián, Gipuzkoa (ES)
(74) Representative: ABG Intellectual Property Law, S.L.

(57) **Abstract**

The present invention relates to a cell culture device suitable as an organ-on-chip device. The device is characterized by having two fluid circuits, one to allow seeding a culture chamber with cells, and a second circuit to maintain a continuous feed of the culture medium until the seeded cells have a suitable degree of growth. Particularly, the device is adapted for cell culture to be carried out in a hydrogel matrix that allows simulation, for example, of the development of tumors in a biological organ.

## Description

### OBJECT OF THE INVENTION

The present invention relates to a cell culture device suitable as an organ-on-chip device. The device is characterized by having two fluid circuits, one to allow seeding a culture chamber with cells and a second circuit to maintain a continuous feed of the culture medium, allowing the seeded cells to maintain a suitable rate of growth and achieve confluence. Particularly, a three-dimensional model simulating the microenvironment and the key functional aspects of an organ is developed.

The expression "culture medium" must be interpreted as a fluid containing the nutrients and factors essential for cell maintenance and growth, allowing the circulation of vital substances and the suitable exchange of gases and waste between the cultured cells.

Particularly, the device is adapted for cell culture to be carried out in a hydrogel matrix that mimics the extracellular matrix (ECM) and allows to mimic, for example, of the development of tumors in a biological organ *in vitro.*

The device is also characterized by its capacity to be easily extracted from the organ for study.

### BACKGROUND OF THE INVENTION

One of the most intensively developed fields of the art, and more specifically in biology, is that of devices that simulate cell growth conditions similar to real conditions in an organ. In this field of biotechnology, cell culture media have been developed to allow the growth and maintenance of cells under controlled conditions outside a living organism.

Conventionally, cells are grown in Petri dishes or culture flasks in liquid or gel media, providing the necessary nutrients and environmental conditions such as suitable temperature and CO₂ levels. However, these methods have inherent limitations, such as the inability to accurately replicate the complex physiological conditions experienced by cells within a human or animal body.

Given this limitation, advanced devices have been developed that allow the creation of more controlled microenvironments, known as extracorporeal culture systems, which provide better conditions for the study of cell behavior in environments more similar to biological environments.

Notable among these devices are those known as "bioreactors," which provide dynamic conditions, such as constant nutrient flow and waste removal, partially simulating physiological functions.

The most recent development in this area has been the so-called "organ-on-chip," a device that incorporates one or more cell types into three-dimensional microstructures with characteristics designed to more closely replicate the interaction between tissues and organs in the body.

The main objective of organ-on-chip devices is to recreate, on a reduced scale, the behavior and functions of entire organs, facilitating research in pharmacology, toxicology and personalized medicine without resorting to the direct use of animals or humans.

These miniaturized devices integrate microfluidic structures or even sensors, and allow real-time analysis of cellular responses under controlled conditions that imitate the real physiological environment.

These devices make it possible to study in detail the biology of a tumor such as, for example, cholangiocarcinoma tumor (CCA) by studying its biology in detail, as well as to test new anti-tumor drugs. For this purpose, animal models, which are expensive and do not fully reflect reality due to the differences between animals and humans, are currently used. Moreover, this type of technique avoids the ethical problems associated with the use of animals.

Notwithstanding the above, known organ-on-chip devices allow the feeding of a culture medium by means of reservoirs that are in contact, directly or indirectly, with the cells and reproduce a static behavior. In these known devices, for example, the feed ends up being consumed over time and, more importantly, the behavior is far from the dynamic behavior that would correspond to that shown when in a body. These limitations observed in prior art devices prevent them from suitably mimicking physiological conditions.

Likewise, they are devices that must be previously seeded in order to subsequently add the culture media so that they can subsequently pass to a growth phase. This first seeding step is a manual step that is often carried out outside the device and must subsequently be manually integrated into the device.

### DESCRIPTION OF THE INVENTION

The invention according to any of the embodiments overcomes the drawbacks identified by means of a structure of the chip, according to a first aspect of the invention, which allows the automation both of the initial phase in which a culture chamber is seeded with cells and of the post-culture phase in which, with the device in the operational form, physiological conditions are mimicked suitably with a live culture.

The expression "in the operational mode" is to be interpreted to mean the conditions in which the device operates when in use. It is in use conditions when the device contains elements such as a hydrogel or fluids flowing through various microfluidic conduits.

The first aspect of the invention relates to a device suitable as an organ-on-chip device, and more specifically adapted as an organ-on-chip device, comprising:
- *a body comprising a first base configured, in the operational mode, to be the lower base, and a second base located opposite the first base, configured, in the operational mode, to be the upper base, wherein the body comprises:*
   *a culture chamber;*
   *a first inlet port in fluid communication with the culture chamber intended for introducing a culture medium;*
   *a second inlet port intended for seeding the culture chamber with cells, the second port being in fluid communication with the culture chamber and communicated with the interposition of a first membrane having a first porosity configured for the passage of the cells to be cultured and configured to not allow the passage of cell aggregates; and*
   *wherein the culture chamber is in fluid communication with an outlet port with the interposition of a second membrane having a second porosity less than the first porosity, configured to prevent the passage of the cells to be cultured, allowing the passage of the culture medium.*

Particularly, when it is indicated that the device comprises a first base configured, in the operational mode, to be the lower base, and a second base located opposite the first base, configured, in the operational mode, to be the upper base, it is to be interpreted to mean that, in use conditions, the first base is in a lower position with respect to the second base considering the direction imposed by the action of gravity.

Preferably, the first base is the base that serves as a support in the lower area of the device and the second base is located in the highest area. These aspects will be relevant in certain examples given that the action of gravity intervenes in the movement of the fluid. Preferably, applicable to all the embodiments described, the fluid connectors are located in the second base.

The device comprises a culture chamber, which is the chamber containing a hydrogel in operational conditions that serves as a supporting structure for the cells while they are being cultured.

In use conditions the device shows two different situations, a first situation in which the culture chamber, containing a hydrogel, is seeded with new cells. The second situation is after the first situation and is that in which the hydrogel which already contains the cells to be cultured, is fed continuously by a culture medium.

The first port serves for this second situation, being a port intended for the entry of a culture medium which is introduced in the device, passing through the volume delimited by the culture chamber and exiting through an outlet port. The inclusion of propulsion means that ensure the uninterrupted entry of the culture medium allows the cells to be fed continuously without variation in the composition of the culture medium, for example, because the nutrients are depleted. These can be replaced continuously from the outside.

A second use of the first port is the introduction of drugs which allow observation of their activity with respect to the organ *in vitro* when it has already been developed. In addition to the culture medium, the fluid incorporates the drug to be tested.

The second port intervenes in the first situation in which the culture chamber holds a hydrogel. Due to the action of gravity, in the operational form, the hydrogel is maintained in the lower area of the culture chamber. The second port allows the entry of cells intended for individually seeding the hydrogel, preventing the existence of cell aggregates from the start which could distort the behavior during growth when the purpose is to observe the evolution from the start of a tumor structure.

For this purpose, the device comprises two membranes, a first membrane and a second membrane.

The first membrane has a porosity suitably selected to allow the passage of individual cells intended for seeding the hydrogel, but it does not allow the passage of cell aggregates which, clustered together, acquire a characteristic larger diameter. It should be noted that the terms cluster or clustering and accumulation or accumulating are considered synonymous in this description and refer to the coming together of two or more cells forming a particle.

Characteristic diameter is understood to mean a distance representative of the dimensions of the cell aggregate. The fact that the porosity of the first membrane is less than the characteristic diameter of a cell aggregate means that the pore size of the membrane is smaller than the dimensions that the aggregate acquires and therefore prevents the passage said aggregate through the membrane.

One skilled in the art familiar with the characteristics of the cells to be cultured would know to select the pore size of the membrane based on the cell size to allow the passage of the cell but not the passage of an aggregate.

Likewise, at the outlet, the device comprises a second membrane with a pore size that prevents the passage of individual cells. This membrane allows the exit of the culture medium to maintain the continuous feed but prevents the loss of cells that could exit the device.

An embodiment applicable to the described device is described, *wherein the first membrane has a first porosity between 10 µm and 30 µm, and more preferably between 15 µm and 25 µm, and more preferably between 18 µm and 22 µm, and more preferably of 20 µm.*

According to this embodiment, the membrane ranges allow causing the seeding of the culture chamber with individual cells and not with cell clusters, specifically for the culture of cholangiocarcinoma (CCA) cells.

An embodiment applicable to the device described in any of the above examples is described, *wherein the second membrane has a second porosity between 0.2 µm and 0.8 µm, and more preferably between 0.3 µm and 0.6 µm, and more preferably between 0.3 and 0.5 µm, and more preferably of 0.4 µm.*

This second membrane is what prevents the exit of the individual cells cultured in the culture chamber, allowing the exit of the culture medium used in the continuous feed flow. These dimensions are also particularly suitable for the culture of cholangiocarcinoma (CCA) cells.

An embodiment applicable to the device described in any of the above examples is described, *wherein the fluid communication between the culture chamber and the outlet port has the outlet from the culture chamber proximal to one of the bases, either the first base or the second base, and the fluid communication between the culture chamber and the first port has the inlet to the culture chamber proximal to the base opposite the base proximal to the outlet.*

In the context of the present invention, the term *proximal* is to be interpreted to mean the portion or area closest to the predetermined reference point, usually the first base or the second base. The term is used in opposition to *distal,* which refers to the portions or areas farthest away from said reference point, particularly according to a certain direction. This interpretation is applicable for any component or element mentioned in the present patent in relation to its proximity to other components or structures. Specifically, a fluid inlet is proximal to a base when its position is very close to the base or is in contact with the base. A microfluidic conduit is understood to be very close to a reference, for example, to a base, when it shows a distance between the closest points less than twice a characteristic measurement of the section, for example, its hydraulic diameter.

The hydraulic diameter of a microfluidic conduit is defined as the ratio of four times the area (A) of the section divided by the wetted perimeter (p) of said section, that is, (4A/p). Wetted perimeter is understood to mean the perimeter of the section that is in contact with the fluid in the operational mode.

It is specified that the distance of a structure, for example, a fluid conduit, is established between "the closest points" to the reference and the reference, for example to one of the bases, given that this structure will contain more or less distant points. For example, a conduit having a circular section that runs parallel to a flat base will have a line of the perimeter of the conduit as a line of points closer to the base, and another line, diametrically opposite the previous one, as a line of points farther away from the base. In this case, it is the line of points closer to the base and their distance from same that serves as a criterion of closeness and of measurement of the distance. Likewise, by way of example, if a conduit is made on the surface of a base in in the form of a groove, and the conduit is constructed by the closing established by a laminar covering, the distance from the conduit to the base will be considered to be zero.

Going back to the embodiment, the flow inlet and outlet of the culture chamber are established at points close to opposite bases. Given that the first base in the operational mode is the lower base and the second base is the upper base, it implies that the flow has either an upward movement or a downward movement. In either case, this upward or downward movement requires going through the culture chamber vertically.

An embodiment applicable to the device described in any of the above examples is described, *wherein the outlet from the culture chamber is at a location opposite, with respect to the projection onto one of the bases, the location of the inlet into the culture chamber.*

According to this embodiment, the inlet and outlet of the culture chamber are at opposite locations regardless of the height thereof considering the orientation in the operational mode. This condition requires the incoming fluid to go through the entire culture chamber according to a direction parallel to one of the bases, preferably a horizontal direction, regardless of whether it has an upward or downward movement. This condition is to be interpreted to mean that one of the bases or the two bases are defined by a plane and the projection is established according to a direction perpendicular to said plane. In a preferred example, the plane of projection is horizontal and the direction of projection is vertical.

When this condition at the opposite position between the fluid inlet and the fluid outlet in the culture chamber is combined with the previous condition of showing opposite positions also in the vertical direction, then the flow that is established inside the culture chamber has optimal conditions to go through most of the hydrogel in the operational mode.

An embodiment applicable to the device described in any of the above examples is described, *wherein the fluid communication between the culture chamber and the first inlet port has the inlet to the culture chamber proximal to the first base.*

This embodiment is of particular interest when the outlet port is located proximal to the second base, in the upper part, which causes the incoming flow to horizontally cross the culture chamber from one end to the other opposite end, and also according to the vertical direction. This configuration allows an optimal feed of the entire volume of the culture chamber.

An embodiment applicable to the device described in any of the above examples is described, *wherein the fluid communication between the culture chamber and the second inlet port has the inlet to the culture chamber proximal to the second base.*

According to this embodiment, the feed of the culture chamber in the initial phase with cells introduced in the operational mode through the second port is through the second base and therefore through the upper part.

This feed from the upper part allows the cells that are introduced to be under the action of gravity, causing a tendency to descend which favors colonization of the entire hydrogel volume by the cells.

Additionally, if the inlet into the culture chamber coming from the first port is located proximal to the first base, then both inlets are spaced apart and separated from one another, so that completely independent flows are established up to the inlet into the culture chamber.

An embodiment applicable to the device described in any of the above examples is described, *wherein the first membrane, the second membrane, or both membranes are housed in an intermediate chamber, wherein the intermediate chamber has its fluid feed in the part proximal to the first base and the fluid outlet in the part proximal to the second base.*

In this embodiment, the membrane is in an intermediate chamber. This chamber increases the area that the membrane through which the fluid transporting cells is to pass can have to prevent possible saturations thereof. Additionally, in this embodiment the entry occurs in the lower area, so any particle which tends to descend onto the membrane due to gravity is eliminated with the fluid in the operational mode. Likewise, in the lower part, if the passage of a cell through the membrane is prevented, the action of gravity will cause the membrane to descend, releasing the filtration surface. The conditions of the flow when it reaches the membrane are therefore homogeneous, and it is the membrane that furthermore imposes said flow being distributed along the area thereof, fomenting said homogeneity to a greater extent.

An embodiment applicable to the device described in the above example is described, where
- *between the fluid feed and the fluid outlet of the intermediate chamber there is a seat in the form of perimeter step to support the membrane;*
- *the membrane is fixed to the seat;*
- *the membrane divides the interior space of the intermediate chamber into two smaller spaces.*

According to the structure specific of the intermediate chamber according to this embodiment, the seat allows the fixing of the membrane, mainly along its perimeter. A preferred configuration of the seat is to arrange it contained in a plane parallel to the first base so that, in the operational mode, it has a horizontal orientation, which facilitates the manufacture and fixing of the membrane.

A preferred configuration of the seat is in the form of a perimeter step.

The membrane, fixed to the seat, define a space on both sides. That is, the membrane shows a sub-chamber under the membrane functioning as a collection chamber which allows the fluid to be distributed along the area of the membrane, and a sub-chamber on the membrane functioning as a collection chamber which allows the entire filtered fluid to be received so as to drive it to the outlet of the intermediate chamber.

An embodiment applicable to the device described in the above example is described, *wherein the membrane is parallel to the first base.*

The condition of the membrane being parallel to the first base, since in the operational mode it is the base that serves as a support, even though other elements such as a plate or a sheet are interposed, results in an also horizontal position of the membrane. This position causes all the points of the membrane to be at the same height conditions, which makes the filtration process more homogeneous.

An embodiment applicable to the device described in any of the above examples is described, *wherein the body is configured as a plate, and the chambers and*/*or conduits are open towards the first base, towards the second base or towards both bases, and wherein:*
- *a first sheet or plate is fixed to the first base, and*
- *a second sheet or plate is fixed to the second base,*
*wherein both sheets or plates close the intermediate chambers and conduits proximal to same, and the first sheet or plate additionally closes the access to the culture chamber through the first base.*

The term sheet or plate is interpreted to mean a structure extending along two dimensions which predominate over the remaining third dimension. The sheet is considered a thinner structure which is capable of sustaining strain which allows the warping thereof or even a double curvature, whereas the term plate is considered, within the context of the theory of elasticity, that a plate is a structural element designed to withstand loads applied perpendicular to its plane, with the stress mainly being distributed in the form of bending and shear stress. That is, the plate is designed to resist loads distributed on its surface, and the internal stress is generated in two dimensions, in contrast with one-dimensional structural elements. This does not exclude weak plates where the levels of strain allowed are very small before reaching breakage, for example fragile glass sheets.

When the configuration of the body of the device is in the form of a plate, the preferred configuration, it can be manufactured using typical methods, either by molding or by subtractive manufacture, by accessing through the first base, the second base, or both bases.

In this case, the body with a plate structure has preferably larger thickness than the plates which are fixed to the first base and to the second base.

With respect to the chambers, the preferred configuration is proximal to one of the bases since the plate that configures the body establishes an open space in said base that is subsequently closed by means of the sheet or plate that is fixed to that same base.

With respect to the conduits, they are configured as channels open towards one of the bases. The sheet or plate which is fixed to the base on which the channel is open configures the conduit.

Lastly, when a conduit is proximal to one of the bases at one end and is proximal to the other base at the other end, a preferred configuration is for it to extend as an open channel in one base from one end and an open channel in the other base from the other end such that the channels are communicated at an intermediate point through a perforation perpendicular to the plate. The channels are closed through the sheet or plate fixed to the base. With this configuration, the channels and the perforation can be configured by molding with simple molds, or if it is manufactured with subtractive machining techniques, they are also accessible by means of a milling operation.

The resulting configuration is such that the seat of the membrane can also be manufactured by any of these means, for example without the need for the use of movable cores, causing the resulting molds or machining operations, if is this the case, to be simpler and to require fewer manufacturing resources and parts.

An embodiment applicable to the device described in the above example is described, wherein:
- *the culture chamber is additionally open through an opening arranged in the second sheet or plate located in the second base;*
- *it comprises a removable sheet or plate configured to close the opening wherein this removable sheet or plate.*

According to this embodiment, the culture chamber is configured to be open through an opening arranged in the second sheet. That is, the second sheet is fixed to the second base but does not close the culture chamber. This allows the interior thereof to be left accessible, for example to extract the cultured organ. According to a preferred example, the opening has the dimensions of the culture chamber so as not to cause an area of the sheet or plate to be cantilevered over the culture chamber, preventing the easy removal of the cultured organ.

Additionally, the culture chamber open through the sheet or plate can be closed by means of a third removable sheet or plate, which allows, in operational conditions, to keep the culture chamber closed and in a differentiated form with respect to the conduits or intermediate chambers. The conduits or intermediate chambers are always kept closed by the second sheet or plate attached to the second base, even though this third sheet or plate is removed, leaving the culture chamber open. According to one embodiment, the third sheet is attached to the second sheet, at least temporarily, and preferably by adhesive bonding.

A second aspect of the invention relates to a culture system comprising a device according to any of the examples described above, wherein *the system further comprises first propulsion means configured, in the operational mode, to propel a culture medium, wherein the propulsion means are in fluid connection with the first inlet port and with the outlet port, and to establish a continuous replenishment of the culture medium.*

The system is configured by the device and the first propulsion means which establish a continuous feeding circuit of the device and therefore of the culture chamber contained therein. The first feeding means in the operational mode feed the inlet of the device and receive the outgoing fluid from the outlet port.

According to the preferred mode, the first propulsion means configure a closed circuit.

An embodiment applicable to the system described in the above example is described, *comprising an intermediate reservoir of the culture medium interposed between the first propulsion means and the device to ensure the uninterrupted feed.*

According to this embodiment, the recirculated flow is passed through a reservoir. The reservoir may have a function of supplementing the fluid that has been depleted by consumption or it may have the function of receiving a medium having the substances, serving as food for the cell culture, partially consumed and providing a newly enriched medium. The reservoir may incorporate both options simultaneously. In this case, the term "may" is to be interpreted as being an embodiment option, not merely a possibility subject to uncertainty.

An embodiment applicable to the system described in any of the two examples described above, that is, the example incorporating propulsion means and the example comprising an intermediate reservoir of the culture medium, is described, *further comprising second propulsion means which are configured to feed the second inlet port with a cell carrier medium to populate the culture chamber.*

The system thus completed incorporates second propulsion means which act in a first step intended for seeding a hydrogel with cells by introducing them through the second port.

A third aspect of the invention relates to a culture method using a system such as any of those described above, which comprises:
- *making the open culture chamber accessible through the second base;*
- *introducing a hydrogel in the culture chamber, taking up part of its interior space;*
- *closing the culture chamber with a removable sheet or plate;*
- *introducing a cell culture in suspension by means of the second propulsion means until the cells are incorporated in the hydrogel;*
- *crosslinking the hydrogel and incubating same for a pre-established period of time, preferably at least 24 h*
- *switching on the first propulsion means establishing a continuous feed of the culture medium in the culture chamber and maintaining same until cells grow in the hydrogel.*

The method uses the system comprising a device such as any of those described and at least propulsion means of the culture medium that ensures suitable conditions for the cells on a continuous basis.

In a first step, a hydrogel is introduced into the culture chamber of the device occupying part of its interior space, preferably without reaching the outlet of the chamber reaching the outlet port. In this preferred way, the action of gravity keeps the hydrogel in the culture chamber even if there is a circulating flow of the culture medium.

The culture chamber is closed with a removable sheet or plate so that the culture chamber is only accessible through the inlet ports and exit is only possible through the outlet port.

Once closed, a fluid with cells is circulated through the second port in order to populate the hydrogel with cells. These cells must go through the first membrane to ensure that the hydrogel is seeded with individual cells, and any cell aggregates that appear in the hydrogel are due to subsequent culture and evolution.

Once the hydrogel is seeded with cells, the hydrogel is crosslinked by any of the available methods, for example chemically or by means of light, always choosing a method that is compatible with cell culture, i.e., that does not destroy the cells colonizing the hydrogel.

The hydrogel crosslinking process causes at least partial polymerization, causing a structural support that simulates the structure of a biological tissue. Preferably, it is considered that the crosslinking and seeding time of the cells in the hydrogel should be at least 24 hours.

Once the hydrogel has been crosslinked, the first propulsion means are switched on, establishing a circulation of the culture media that ensures cell viability.

The time that the culture remains fed is the time necessary for the proliferation of the cells in the hydrogel to occur and to allow their subsequent study. For example, it is possible to subsequently remove the removable sheet or plate that closes the culture chamber to remove the cultured organ formed by the crosslinked hydrogel with the cell culture for further study.

Preferably, in all the described examples both the main body of the device and the sheets or plates are transparent to allow an inspection of the evolution of the experiment or even measurement and photography with more advanced means in a non-intrusive way.

After these steps that allow the proliferation of the cells and formation of the organ, the removable sheet or plate that closes the culture chamber is removed for the extraction of the hydrogel and the cultured cells and this allows an analysis directly on the structures and cells that have grown on the hydrogel block that simulates the function of a biological organ.

One skilled in the art will know which hydrogel to choose in the present invention; it will be any hydrogel suitable for cell culture and the crosslinking method of which is compatible with cell viability. A hydrogel is a translucent semi-solid formulation comprising at least a gelling portion and an aqueous fluid portion, both being homogeneously distributed. The hydrogel may be synthetic or natural and the crosslinking may be irreversible or reversible. Non-limiting examples of hydrogel include collagen, alginate, gelatin, Matrigel, hyaluronic acid, albumin, agarose, chitosan, heparin, fibrin, polyacrylic acid (PAA), and polyacrylates such as polyvinyl acid (PVA), polyethylene glycols (PEGs), and combinations or derivatives thereof.

According to the manufacturer, Matrigel (Corning BioCoat^{®}) is a soluble basal decellularized matrix extracted from the EHS (Engel breth-Holm-Swarm) tumor that, when solidified, forms a structure equivalent to a basement membrane in terms of composition, structure, and physical properties. The most important components of Matrigel are laminin, collagen IV, entactin, and heparan sulfate proteoglycan.

In a particular embodiment, the hydrogel is selected from collagen, alginate, gelatin, Matrigel, hyaluronic acid, albumin, agarose, chitosan, heparin, fibrin, and combinations or derivatives thereof, preferably collagen, Matrigel, and combinations or derivatives thereof, more preferably a mixture of collagen and Matrigel, and even more preferably an 80:20 (w/w) mixture of collagen and Matrigel.

One skilled in the art will also know which method to choose for polymerizing the hydrogel. In this context, the terms "crosslinking", "polymerizing", and "gelling" are used synonymously. Non-limiting examples of methods for crosslinking the hydrogel include irradiation with a wavelength of less than 800 nm, temperature change, pH change, addition of crosslinking agents including metallic salts such as CaClz, polymeric conjugation, enzymatic catalysis, or combinations thereof. Irradiation presupposes the presence of photosensitive agents in the hydrogel, and comprises preferably visible and UV light irradiation, more preferably UV light irradiation.

As mentioned above, analysis of cellular responses in cholangiocarcinoma (CCA) tumor, both intrahepatic CCA and extrahepatic CCA, has been identified as a possible embodiment under the conditions of use of the device. In this embodiment the device may be used, without limitation, under conditions suitable for the culture of one or more of the Huh28 and HuCC-T1 cell lines, in the case of intrahepatic CCA, or one or more of the EGl11, TFK1, and WITT cell lines in the case of extrahepatic CCA. Also, the use of the device for the analysis of CCA tumor cell responses may include, in addition to the cell lines mentioned above, other cell types present in the tumor microenvironment, such as and not limited to these examples, cancer-associated fibroblasts (CAFs) and macrophages derived from the THP-1 monocyte cell line.

### DESCRIPTION OF THE DRAWINGS

These and other features and advantages of the invention will become more apparent from the following detailed description of a preferred embodiment, given only by way of illustrative and non-limiting example, in reference to the attached figures.
- Figure 1: This figure shows a perspective view of an embodiment of the main body of the device. The perspective view allows the concealed chambers and conduits to be observed using grey lines and discontinuous lines.
- Figures 2A, 2B: These figures show the same embodiment in an elevational view and plan view, respectively.
- Figure 3A: This figure shows the same embodiment in a perspective view as in Figure 1, where now the parts of the concealed chambers and conduits are not shown to simplify the image, and where a first plate intended to be fixed to the first base, a second plate intended to be fixed to the second base, and a removable third plate intended to cover the second plate have been depicted schematically and with a discontinuous line. All this is shown in an exploded perspective view.
- Figure 3B: This figure shows the same view shown in Figure 3A where the three plates are fixed in their final position, except the removable plate, which is shown in the open position, hinged on one of the sides to show the closing by the rotation thereof, closing the opening that provides access to the culture chamber.
- Figure 4: This figure shows a system in which propulsion means feed the device shown in the previous figures. The closed circuit includes the reservoir.

### DETAILED DESCRIPTION OF THE INVENTION

According to the first inventive aspect, the present invention relates to a cell culture device.

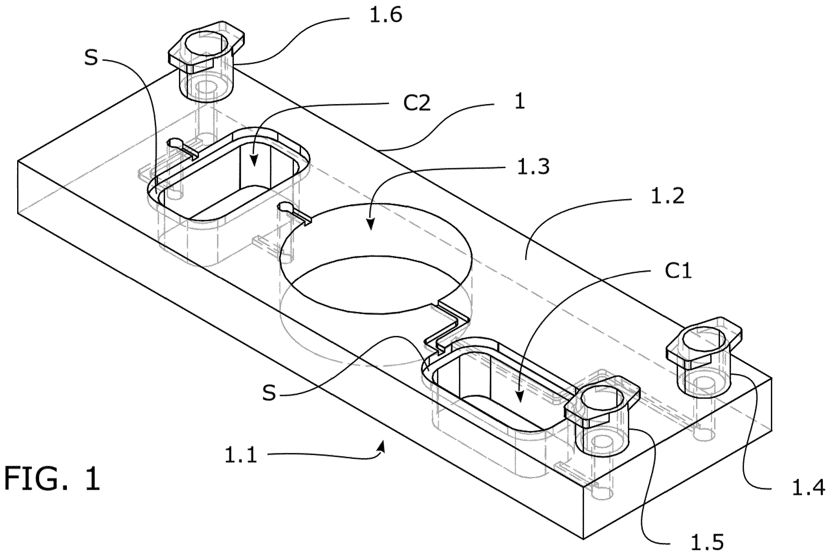

Figure 1 shows a specific embodiment, particularly the main body (1) configured in the form of a thick plate, with a first base (1.1), the lower base, and a second base (1.2), the upper base. Both bases (1.1, 1.2) in this embodiment are flat and parallel.

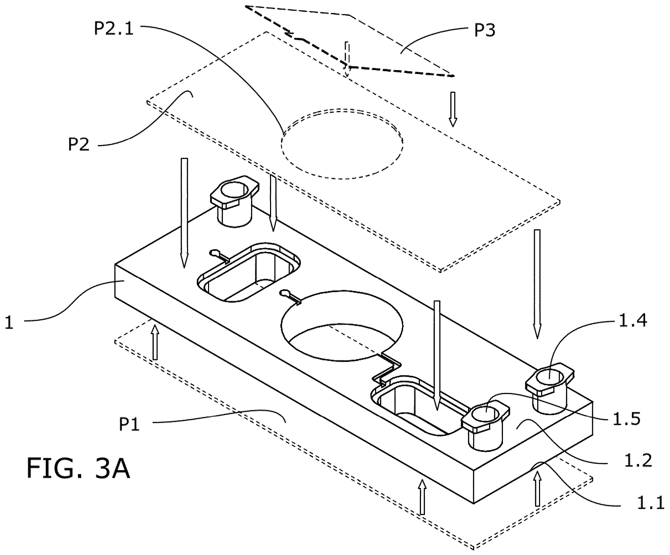

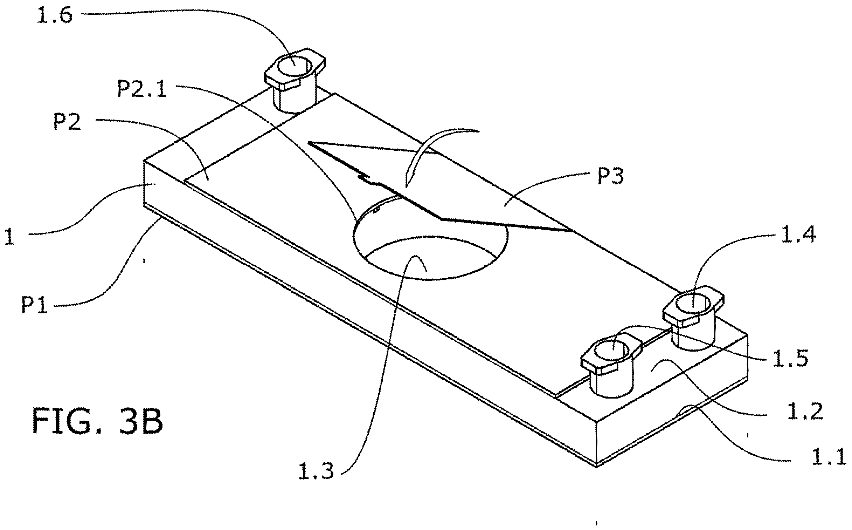

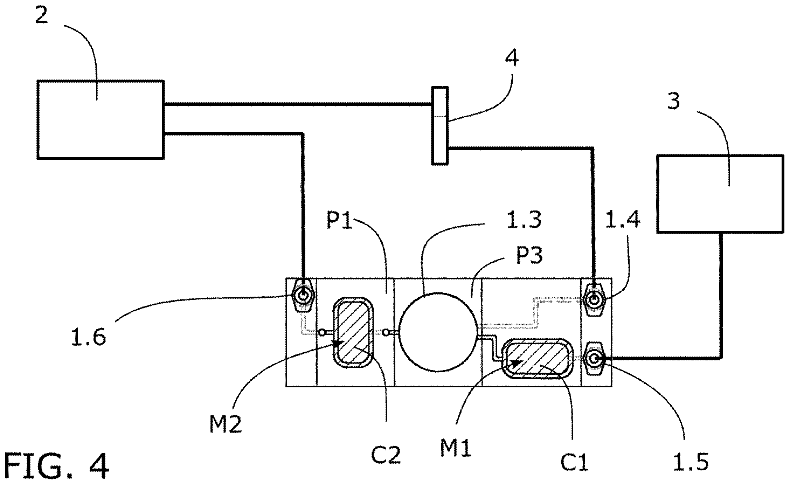

The main body comprises a culture chamber (1.3) which, in this embodiment, is a cylindrical through cavity, that is, it is accessible from both bases (1.1, 1.2). In this figure, the culture chamber (1.3) appears as an empty space given that it becomes a chamber once the first base (1.1) is closed by a first plate (P1) and the second base is closed by a second plate (P2) and a removable third plate (P3), as will be described in reference to Figures 3 and 4. The same occurs with the different conduits. In Figures 1, 2, and 3 they appear in most of their extension as channels open either towards the first base (1.1) or towards the second base (1.2) given that they are conduits when they are covered by one of the plates (P1, P2).

This configuration of the body (1) allows a manufacture by molding or by subtractive manufacture, for example by milling. In the case of manufacture by molding, the molds are closed in the direction perpendicular to the bases (1.1, 1.2), so the shape allows the configuration of voids and channels which then give rise to the closed chambers and conduits when the plates (P1, P2, P3) are fixed.

The culture chamber (1.3) can be fed either by means of a first port (1.4) or by means of a second port (1.5). The first port (1.4) is intended for the continuous feed of the culture medium which allows cell culture and growth, and the second port (1.5) is intended for the initial feed of a flow with cells to seed with cells a hydrogel housed in the operational form in the culture chamber (1.3).

The exit of any fluid from the culture chamber (1.3) is established, in this example, through a single outlet shown by means of an outlet port (1.6) located on the side opposite the inlet ports (1.4, 1.5).

The three ports (1.4, 1.5, 1.6) show standard microfluidic connectors which allow the connection with standard connectors located at the ends of flexible conduits that will form the system by incorporating propulsion means.

In this embodiment, the three ports are also located on the side of the second base (1.2), that is, located in the upper part since the first base (1.1) serves as a support base and, following the direction imposed by the action of gravity, is located in lower operational position. All relative positional references such as up, down, horizontal, vertical, will be provided with respect to the action of gravity taken as a reference when the first base (1.1) is the support base and is arranged horizontally.

In the operational mode, the first port (1.4) receives the culture medium and is connected with the culture chamber (1.3) by means of a first segment that descends down to the first base (1.1) and is extended by means of a second segment parallel to the first base (1.1) to access the culture chamber (1.3) in the lower portion.

The outlet is established at a point located on the opposite side with respect to the plan projection as shown in Figure 3, only slightly displaced, and also in the upper, that is, opposite, part in the vertical direction. With this arrangement of the outlet, the fluid of the culture medium goes through the entire culture chamber (1.3) both horizontally and vertically, optimizing the feed of the entire volume of the culture chamber (1.3).

In the operational mode, the second port (1.5) receives a fluid with cells intended to populate the hydrogel which is housed in the culture chamber (1.3), and by means of a first segment, descends down to the first base (1.1) to thus feed a first intermediate chamber (C1) which in turn is communicated with the culture chamber (1.3). The communication between the first intermediate chamber (C1) and the culture chamber (1.3) is established parallel to the second base (1.2) and therefore through the upper part, which allows the cells entering the culture chamber (1.3) to descend by gravity, seeding the entire volume of the hydrogel.

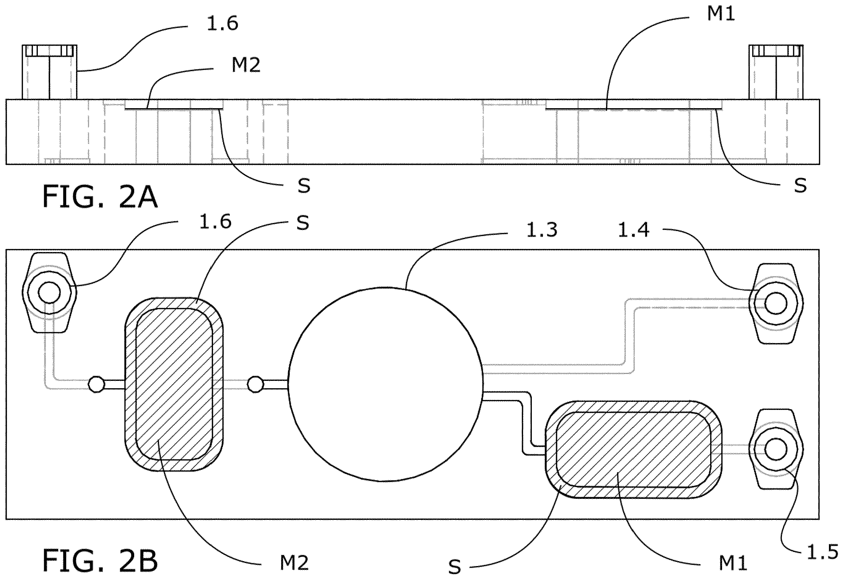

The first intermediate chamber (C1), as shown both in Figure 2 in an elevational view and in Figure 3 in a plan view of the body (1) of the device, shows a first space or lower sub-chamber accessible from the first base (1.1), and a second space or upper sub-chamber having a larger area in plan projection than the lower sub-chamber. The change in area in plan projection results in a perimeter step which allows a seat (S) to be configured for a first membrane (M1) with a porosity such that it allows the passage of individual cells but does not allow the passage of cell aggregates.

The structure of this first intermediate chamber (C1) which is interposed between the second port (1.5) and the culture chamber (1.3) is like a second intermediate chamber (C2) interposed between the outlet of the culture chamber (1.3) and the outlet port (1.6).

In the second intermediate chamber (C2), the membrane identified as the second membrane (M2) has a porosity less than the first membrane (M1) configured to not allow the exit of cells coming from the culture chamber (1.3).

Both the first intermediate chamber (C1) and the second intermediate chamber (C2) show a configuration which, according to the configuration in a plan view like the one shown in Figure 2B, is rectangular with rounded vertexes. The first intermediate chamber (C1) has the inlet and outlet on opposite smaller sides of this rectangular configuration, whereas the second intermediate chamber (C2) has the inlet and outlet on opposite larger sides of the rectangular configuration.

Both in the inlet and in the outlet of the second intermediate chamber (C2), the conduits transfer fluid from the upper base to the lower base, a first inlet conduit connecting the culture chamber (1.3) with the inlet of the second intermediate chamber (C2), and a second outlet conduit connecting the culture chamber (1.3) with the lower segment that opens into the outlet port (1.6). This transfer from one base to another, from the upper or second base (1.2) or to the lower or first base (1.1), is carried out by means of a structure with three parts, i.e., a first part formed by a channel open towards the second base (1.2) and closed by the second plate (P2), a second part formed by a circular through perforation communicating the second base (1.2) with the first base (1.1), and finally by means of a third part formed by a channel open towards the first base (1.1) which is closed by the first plate (P1). This structure with three parts allows the configuration of a conduit that can be readily manufactured either by molding or by machining with established movements in the direction perpendicular to the body.

Figure 3A shows an exploded perspective view which also shows, in addition to the body (1) described together with its inlet ports (1.4) and outlet port (1.6), the first plate (P1) in the lower position and the second plate (P2) in the upper position, where the hollow arrows indicate the direction of approach until contacting the respective bases (1.1, 1.2) to which they are fixed.

Likewise, a removable third plate (P3) is shown. The second plate (P2) comprises an opening (P2.1) coinciding, according to a plan projection and when the second plate (P2) is fixed on the second base (1.2) of the body (1), with the shape in a plan view of the culture chamber (1.3). Therefore, the opening (P2.1) establishes continuity to the side walls of the culture chamber (1.3). This opening (P2.1) allows access to the interior of the culture chamber (1.3) except when the removable plate (P3) is closing the opening (P2.1). The closing is established when the device contains the hydrogel and is in the operational mode, serving as support for the different steps of culturing.

The three plates (P1, P2, P3) are shown in discontinuous lines.

Figure 3B shows the same perspective view as Figure 3A where the three plates (P1, P2, P3) are now shown with a continuous line in a position close to the operational position. That is, the first plate (P1) and the second plate (P2) are located in the first base (1.1) and the second base (1.2), respectively, closing intermediate chambers (C1, C2) and channels that are open to configure conduits.

Furthermore, the opening (P2.1) of the second plate (P2) which provides access to the interior of the culture chamber (1.3) is partially covered by the removable plate (P3).

In the figure it is shown supported on an edge, leaving the interior of the culture chamber (1.3) partially accessible. Although this removable plate (P3) is shown to be hinged, it can be fixed to the second plate (P2) by means of an approach movement which does not have to be the result of a hinged movement.

Likewise, although the removable plate (P3) is shown to be opaque, according to a preferred example it is transparent and allows visual access to the interior of the culture chamber (1.3). According to a preferred example, both the three plates (P1, P2, P3) and the main body (1) are transparent to allow inspection of the interior of both the culture chamber (1.3) and other chambers (C1, C2) and conduits.

Figure 3B also shows the configuration of the device if the removable plate (P3) is removed for the purpose of extracting the hydrogel with the cells as they have evolved throughout the feeding and proliferation process, giving rise to a biological organ.

Figure 4 is a schematic figure showing an embodiment of the system using the device described in the preceding figures.

The system incorporates propulsion means (2) feeding the first inlet port (1.4) with a culture medium. The culture medium is transferred through a conduit of the device to the culture chamber (1.3) where there is a hydrogel on the which the cells to be cultured are fixed and, after passing through the culture chamber (1.3), to ensure the viability of the cells, exits until reaching the outlet port (1.6) passing through the second intermediate chamber (C2). The passage through the second intermediate chamber (C2) imposes the culture medium having to pass through the second membrane that prevents the passage of any cell migrating from the culture chamber (1.3) so that said cell does not reach the outlet port (1.6).

The culture medium recovered through the outlet port (1.6) is again propelled by the propulsion means (2), maintaining continuous circulation. According to this embodiment, the culture medium passes through a reservoir (4) before it is introduced in the first inlet port (1.4), such that it ensures that the culture medium is not depleted and maintains the components necessary to ensure the viability of the culture.

This same Figure 4 shows second propulsion means (3) intended to ensure the introduction of a cell carrier fluid intended for seeding the culture chamber (1.3) at the beginning of the entire process. These second propulsion means (3) are in fluid communication with the second inlet port (1.5) which in turn establishes fluid communication with the inlet into the first intermediate chamber (C1). This first intermediate chamber (C1) is responsible for establishing a barrier against the passage of cell aggregates, ensuring passage only for individual cells that will seed the hydrogel housed in the culture chamber (1.3) in the operational mode.

In use conditions, the method using this system starts by having the culture chamber (1.3) open, without the removable plate (P3), to introduce a hydrogel, taking up part of its interior space. In this embodiment, the hydrogel fills the culture chamber (1.3) without reaching the height of the outlet that is located proximal to the second plate (P2) to prevent the hydrogel from exiting or blocking the outlet of the culture chamber (1.3).

Once the hydrogel is incorporated, the culture chamber (1.3) is closed with a removable sheet or plate (P3).

By switching on the second propulsion means, a cell culture in suspension is introduced until the cells are incorporated in the hydrogel of the culture chamber (1.3).

The following step comprises crosslinking the hydrogel and incubating same for a pre-established period of time, preferably at least 24 h. In this hydrogel crosslinking and seeding period, at least partial polymerization of the hydrogel is caused, forming a crosslinking of the hydrogel chains, providing structural properties to the hydrogel volume that are similar to those of a biological tissue. It is in the hydrogel volume where the cells will proliferate and form structures for example similar to tumor structures. The crosslinking process must be such that it ensures the viability of the cells that are seeded.

Once the hydrogel is crosslinked, the first propulsion means (2) are switched on, establishing a continuous feed of the culture medium in the culture chamber (1.3). These propulsion means (2) remain on until complete growth and evolution of the cell culture in the hydrogel.

In this embodiment, when the components of the device are transparent, it is possible to observe the evolution of the organ simulated by the hydrogel and the cell population.

To observe the culture chamber (1.3) and its contents, the propulsion means (3) are switched off, the tubes connected to the inlet/outlet ports (1.4, 1.5, 1.6) are disconnected, and caps are placed in such ports, keeping the contents isolated. Microscopy is carried out in these conditions to observe said contents.

Once the culture period has elapsed, according to an example of the method, the removable sheet or plate (P3) closing the culture chamber (1.3) is removed to extract the hydrogel and the cultured cells. This allows physical handling of the hydrogel as an organ for direct inspection.

## Claims

1. A cell culture device suitable as an organ-on-chip device, and more specifically adapted as an organ-on-chip device, comprising:
- a body (1) comprising a first base (1.1) configured, in the operational mode, to be the lower base, and a second base (1.2) located opposite the first base (1.1), configured, in the operational mode, to be the upper base, wherein the body comprises:
a culture chamber (1.3);
a first inlet port (1.4) in fluid communication with the culture chamber (1.3) intended for introducing a culture medium;
a second inlet port (1.5) intended for seeding the culture chamber (1.3) with cells, the second port (1.5) being in fluid communication with the culture chamber (1.3) and communicated with the interposition of a first membrane (M1) having a first porosity configured for the passage of the cells to be cultured and configured to not allow the passage of cell aggregates; and
wherein the culture chamber (1.3) is in fluid communication with an outlet port (1.6) with the interposition of a second membrane (M2) having a second porosity less than the first porosity, configured to prevent the passage of the cells to be cultured, allowing the passage of the culture medium.

2. The device according to claim 1, wherein the first membrane (M1) has a first porosity between 10 µm and 30 µm, and more preferably between 15 µm and 25 µm, and more preferably between 18 µm and 22 µm, and more preferably of 20 µm.

3. The device according to any of the preceding claims, wherein the second membrane (M2) has a second porosity between 0.2 µm and 0.8 µm, and more preferably between 0.3 µm and 0.6 µm, and more preferably between 0.3 and 0.5 µm, and more preferably of 0.4 µm.

4. The device according to any of the preceding claims, wherein the fluid communication between the culture chamber (1.3) and the outlet port (1.6) has the outlet from the culture chamber (1.3) proximal to one of the bases (1.1), either the first base (1.1) or the second base (1.2), and the fluid communication between the culture chamber (1.3) and the first port (1.4) has the inlet to the culture chamber (1.3) proximal to the base (1.2, 1.1) opposite the base (1.1, 1.2) proximal to the outlet.

5. The device according to any of the preceding claims, wherein the fluid communication between the culture chamber (1.3) and the second inlet port (1.5) has the inlet to the culture chamber (1.3) proximal to the second base (1.2).

6. The device according to any of the preceding claims, wherein the first membrane (M1), the second membrane (M2), or both membranes (M1, M2) are housed in an intermediate chamber (C1, C2), wherein the intermediate chamber (C1, C2) has its fluid feed in the part proximal to the first base (1.1) and the fluid outlet in the part proximal to the second base (1.2).

7. The device according to the preceding claim, wherein
- between the fluid feed and the fluid outlet of the intermediate chamber (C1, C2) there is a seat (S) in the form of a perimeter step to support the membrane (M1, M2);
- the membrane (M1, M2) is fixed to the seat (S);
- the membrane (M1, M2) divides the interior space of the intermediate chamber (C1, C2) into two smaller spaces.

8. The device according to the preceding claim, wherein the membrane (M1, M2) is parallel to the first base (1.1).

9. The device according to any of the claims, wherein the body (1) is configured as a plate, and the chambers and/or conduits are open towards the first base (1.1), towards the second base (1.2), or towards both bases (1.1, 1.2), and wherein:
- a first sheet or plate (P1) is fixed to the first base (1.1), and
- a second sheet or plate (P2) is fixed to the second base (1.2),
wherein both sheets or plates (P1, P2) close the intermediate chambers (C1, C2) and conduits proximal to same, and the first sheet or plate (P1) additionally closes the access to the culture chamber (1.3) through the first base (1.1).

10. The device according to claim 9, wherein:
- the culture chamber (1.3) is additionally open through an opening (P2.1) arranged in the second sheet or plate (P2) located in the second base (1.2);
- it comprises a removable sheet or plate (P3) configured to close the opening (P2.1), wherein this removable sheet or plate (P3).

11. A culture system comprising a device according to any of the preceding claims, further comprising first propulsion means (2) configured, in the operational mode, to propel a culture medium, wherein the propulsion means (2) are in fluid connection with the first inlet port (1.4) and with the outlet port (1.6), and to establish a continuous replenishment of the culture medium.

12. The culture system according to the preceding claim, comprising an intermediate reservoir (4) of the culture medium interposed between the first propulsion means (2) and the device (1) to ensure the uninterrupted feed.

13. The system according to claim 11 or 12, comprising second propulsion means (3) which are configured to feed the second inlet port (1.5) with a cell carrier medium to populate the culture chamber (1.3).

14. A culture method using a system according to any of claims 11 to 13, which comprises:
- making the open culture chamber (1.3) accessible through the second base (1.2);
- introducing a hydrogel in the culture chamber (1.3), taking up part of its interior space;
- closing the culture chamber (1.3) with a removable sheet or plate (P3);
- introducing a cell culture in suspension by means of the second propulsion means (3) until the cells are incorporated in the hydrogel;
- crosslinking the hydrogel and incubating same for a pre-established period of time, preferably at least 24 h;
- switching on the first propulsion means (2), establishing a continuous feed of the culture medium in the culture chamber (1.3) and maintaining same until cells grow in the hydrogel.

15. The method according to claim 14 or 15, wherein the removable sheet or plate (P3) closing the culture chamber (1.3) is removed to extract the hydrogel and the cultured cells.
